# EUROPEAN PATENT APPLICATION

(11) **EP 2 784 515 A2**
(43) Date of publication of application: **01.10.2014**
(21) Application number: 14161525.2
(22) Date of filing: 25.03.2014
(51) Int. Cl.: G01N 35/00

(54) **Sample analysis method, sample analysis system, and recovery method**

(30) Priority: 29.03.2013 JP 2013074795
(71) Applicant: SYSMEX CORPORATION, Kobe-shi, Hyogo 651-0073 (JP)
(72) Inventor: Koeda, Noriaki, Hyogo, 651-0073 (JP)
(74) Representative: HOFFMANN EITLE

(57) **Abstract**

Disclosed is a sample analysis method using a sample measuring apparatus configured to operate under control of a first computer, the method comprising: making the sample measuring apparatus operate under control of a second computer instead of the first computer, the step including downloading, from an external server, a control program for executing a function of causing the sample measuring apparatus to start measurement of a sample, a function of receiving measurement data of the sample from the sample measuring apparatus, and a function of transmitting the received measurement data to the external server, and installing the control program in the second computer; causing the second computer to execute the installed control program to cause the sample measuring apparatus to perform measurement of a sample, and to transmit measurement data received from the sample measuring apparatus to the external server; and causing the external server to execute an analysis program for analyzing the measurement data received from the second computer, to analyze the measurement data and to generate an analysis result.

## Description

### FIELD OF THE INVENTION

The present invention relates to a sample analysis method and a sample analysis system in which, even when abnormality has occurred in a computer associated with a sample measuring apparatus, quick recovery of the sample measuring apparatus and continuation of analysis can be enabled by use of a cloud computer. Further, the present invention relates to a recovery method using a cloud computer.

### BACKGROUND

U.S. Patent Application Publication No. 2012-0141326 discloses a sample processing apparatus which analyzes samples each contained in a sample container. This sample processing apparatus includes a transport unit, a measuring unit, and an information processor. The information processor controls operation of the transport unit and the measuring unit. Further, the information processor performs analysis based on measurement data obtained in the measuring unit, and transmits an analysis result to a host computer.

The information processor of U.S. Patent Application Publication No. 2012-0141326 is implemented by a personal computer. In a hard disk of the information processor, computer programs for controlling operation of the transport unit and the measuring unit, and data to be used for executing the computer programs are stored.

Since the information processor disclosed in U.S. Patent Application Publication No. 2012-0141326 is in charge of control of operation of the transport unit and the measuring unit, if the information processor fails, the entirety of the system goes down. In such a case, it is not easy for a user to replace the information processor. Thus, conventionally, until a repair person of the manufacturer of the apparatus prepares an alternate computer, resumption of measurement has to be waited, which may cause a prolonged down time.

Therefore, an object of the present invention is to enable, when a computer connected to a sample measuring apparatus has failed, the user to quickly resume measurement.

### SUMMARY OF THE PRESENT INVENTION

A first aspect of the present invention is a sample analysis method using a sample measuring apparatus configured to operate under control of a first computer, the first computer having at least a function of causing the sample measuring apparatus to start measurement of a sample, a function of receiving measurement data of the sample from the sample measuring apparatus, and a function of analyzing the received measurement data to generate an analysis result, the sample analysis method comprising: making the sample measuring apparatus operate under control of a second computer instead of the first computer, the step including downloading, from an external server, a control program for executing a function of causing the sample measuring apparatus to start measurement of a sample, a function of receiving measurement data of the sample from the sample measuring apparatus, and a function of transmitting the received measurement data to the external server, and installing the control program in the second computer; causing the second computer to execute the installed control program to cause the sample measuring apparatus to perform measurement of a sample, and to transmit measurement data received from the sample measuring apparatus to the external server; and causing the external server to execute an analysis program for analyzing the measurement data received from the second computer, to analyze the measurement data and to generate an analysis result.

In the first aspect, the method may comprise causing the second computer to refer to the generated analysis result on the external server for displaying it.

In the first aspect, the method may be characterized in that making the sample measuring apparatus to operate under control of the second computer includes establishing a connection between the second computer in which the control program has been installed and the sample measuring apparatus.

In the first aspect, the method may comprise connecting the second computer with a host computer which manages analysis results; and causing the external server to transmit the analysis result generated by the external server to the host computer.

In the first aspect, method may be characterized in that the first computer is capable of storing therein data relating to measurement of a sample or analysis of measurement data, and the method may further comprise causing, while the sample measuring apparatus is capable of operating under control of the first computer, the first computer to transmit the data stored therein to the external server; and causing the external server to store the data therein, to establish data synchronization of the data between the first computer and the external server.

In the first aspect, the method may be characterized in that the data includes information for analysis to be used when the first computer analyzes the measurement data, and the method may comprise: causing the external server to analyze, by using the information for analysis which had been transmitted from the first computer, the measurement data transmitted from the second computer.

In the first aspect, the method may be characterized in that the data includes information for measurement to be used when the first computer controls measurement of a sample performed by the sample measuring apparatus, and the method may comprise: causing the second computer to obtain, from the external server, the information for measurement stored in the external server, and to control, by using the obtained information for measurement, measurement of a sample performed by the sample measuring apparatus.

In the first aspect, the method may be characterized in that causing the external server to provide the second computer with the control program and the information for measurement when the external server receives a request of downloading the control program from the second computer.

In the first aspect, the method may be characterized in that the data includes measurement order information indicating a measurement item to be measured for each of a plurality of samples, and the method may comprise: causing the second computer to cause the sample measuring apparatus to start measurement of each sample based on the measurement order information stored in the external server.

In the first aspect, the method may be characterized in that the sample measuring apparatus includes an ID obtaining part which obtains an ID from a sample container which contains a sample, and the method may comprise: causing the second computer to cause the ID obtaining part of the sample measuring apparatus to obtain an ID, to receive the obtained ID from the sample measuring apparatus, and to transmit the received ID to the external server; causing the external server to search, upon receiving the ID from the second computer, corresponding measurement order information by using the received ID as a key, and to transmit, to the second computer, the corresponding measurement order information or a measurement command based on the corresponding measurement order information; and causing the second computer to control the sample measuring apparatus to start measurement of the sample, based on the received measurement order information or measurement command.

In the first aspect, the method may comprise: causing the external server to transmit the data stored therein to a recovery computer; making the sample measuring apparatus operate under control of the recovery computer instead of the second computer; and causing the recovery computer to analyze measurement data generated in a state where the sample measuring apparatus operates under control of the recovery computer.

In the first aspect, the method may be characterized in that the external server includes: a first server capable of storing therein the data transmitted from the first computer and the data transmitted from the second computer; and a second server which performs data synchronization of the data with the first server, and the method may further comprise: causing, prior to replacement of the second computer with the recovery computer, the second server to stop performing the data synchronization of the data with the first server; causing, upon stop of the data synchronization, the second server to transmit, to the recovery computer, the data transmitted from the first computer or the second computer and stored in the second server; causing, even after the stop of the data synchronization, the first server to update the data stored in the first server, based on data communication with the second computer; and causing, upon completion of transmission of the data stored in the second server to the recovery computer, the first server to transmit difference data, of the data stored in the first server, which has been updated after the stop of the data synchronization with the second server, to the recovery computer.

In the first aspect, the method may be characterized in that the external server is capable of storing therein a data group including the data transmitted from the first computer and other data transmitted from another computer which controls another sample measuring apparatus different from the sample measuring apparatus, and the method may comprise: causing the second computer to transmit, to the external server, data identification information for identifying the data transmitted from the first computer in the data group; and causing the external server to extract the data transmitted from the first computer from the data group, based on the data identification information.

In the first aspect, the method may be characterized in that the external server stores a control program group including the control program used in at least the second computer and a different type of control program for controlling measurement of a sample performed by a sample measuring apparatus of a type different from that of the sample measuring apparatus, and the method may further comprise: causing the second computer to transmit, to the external server, program identification information for identifying the control program in the control program group; and causing the external server to extract the control program from the control program group based on the program identification information, and to transmit the control program to the second computer.

A second aspect of the present invention is a sample analysis system comprising: a sample measuring apparatus including a means for measuring a sample; a computer including a means for executing a control program for causing the sample measuring apparatus to measure a sample, and a communication program for performing communication with an external server; and an external server including a means for storing data and connected to the computer via a network, characterized in that the computer functions as a means for controlling the sample measuring apparatus to measure a sample and a means for receiving measurement data from the sample measuring apparatus by executing the control program, and a means for transmitting the received measurement data to the external server by executing the communication program, the external server functions by executing an analysis program as: a means for analyzing the measurement data to generate an analysis result and a means for storing the generated analysis result in the means for storing, and the computer includes a means for displaying the analysis result by referring the same on the external server.

### ADVANTAGEOUS EFFECTS OF THE INVENTION

According to the present invention described above, even when the computer connected to the sample measuring apparatus has failed, the sample measuring apparatus can be controlled by a computer in which the control program is installed. It is sufficient that the computer in which the control program is installed controls the sample measuring apparatus, and analysis of measurement data can be performed by the external server. Therefore, even when a computer connected to a sample measuring apparatus has failed, the user can quickly resume measurement. Downtime can be greatly reduced.

### BRIEF DESCRIPTION OF THE DRAWINGS

FIG. 1 is a configuration diagram of a sample analysis system.
FIG. 2 is a configuration diagram of a sample analyzer during normal time.
FIG. 3 is a configuration diagram of the sample analyzer while an alternate system is constructed.
FIG. 4A shows stored contents in the sample analysis system.
FIG. 4B shows how modules are allocated during normal time and while the system is distributed.
FIG. 5 is a flow chart of system operation during normal time.
FIG. 6 shows flows of data while an alternate system is constructed.
FIG. 7 is a flow chart of a procedure for constructing the alternate system.
FIG. 8 shows flows of data while the alternate system is in operation.
FIG. 9 is a flow chart of operation performed in the alternate system.
FIG. 10 shows flows of data while an alternate system for recovery is constructed.
FIG. 11 shows flows of data for recovery.
FIG. 12 is a flow chart showing a recovery procedure.
FIG. 13 is a flow chart of a first modification.
FIG. 14 is a flow chart of a second modification.
FIG. 15 is a flow chart of a third modification.

### DETAILED DESCRIPTION OF THE EMBODIMENT

Hereinafter, embodiments of the present invention will be described in detail, with reference to the attached drawings.

### [1. Sample analysis system]

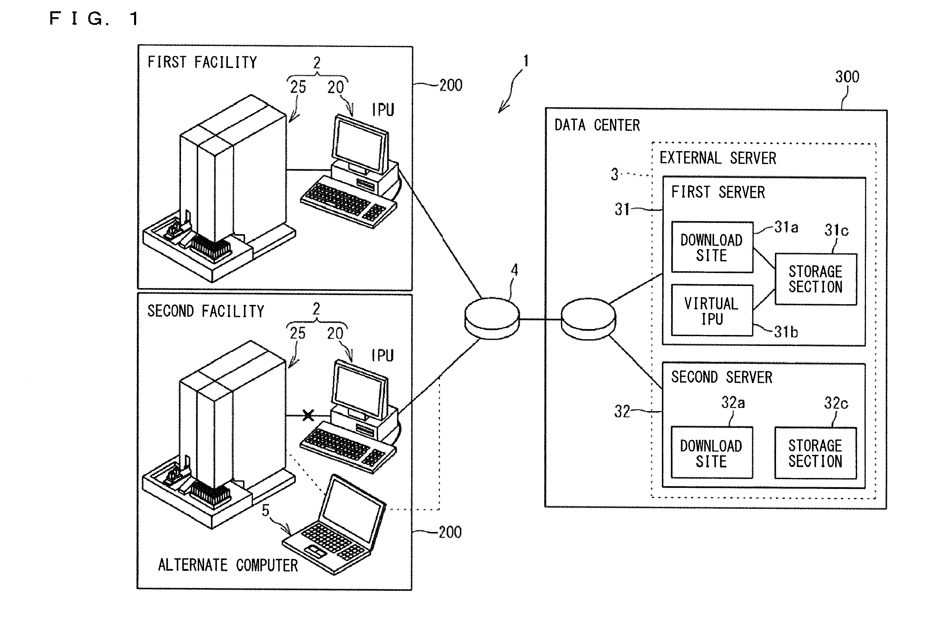

FIG. 1 shows a sample analysis system 1. The sample analysis system 1 includes sample analyzers 2 and an external server 3. Each sample analyzer 2 can communicate with the external server 3 via a network such as Internet 4. The external server 3 is installed in a data center 300. The data center 300 is a facility different from facilities (test institutions, hospitals, and the like) 200 where the sample analyzers 2 are installed. The external server 3 can communicate with the plurality of sample analyzers 2, 2 installed in each of the plurality of facilities 200. The external server 3 includes a first server 31 and a second server 32.

The first server 31 includes a download site 31a which allows download of download data, a virtual IPU 31b, and a storage section 31c in which data and computer programs are stored. It should be noted that the virtual IPU 31b is constructed in the first server as necessary, and the virtual IPU 31b may not always be provided. The second server 32 includes a download site 32a which allows download of download data, and a storage section 32c in which data and computer programs are stored.

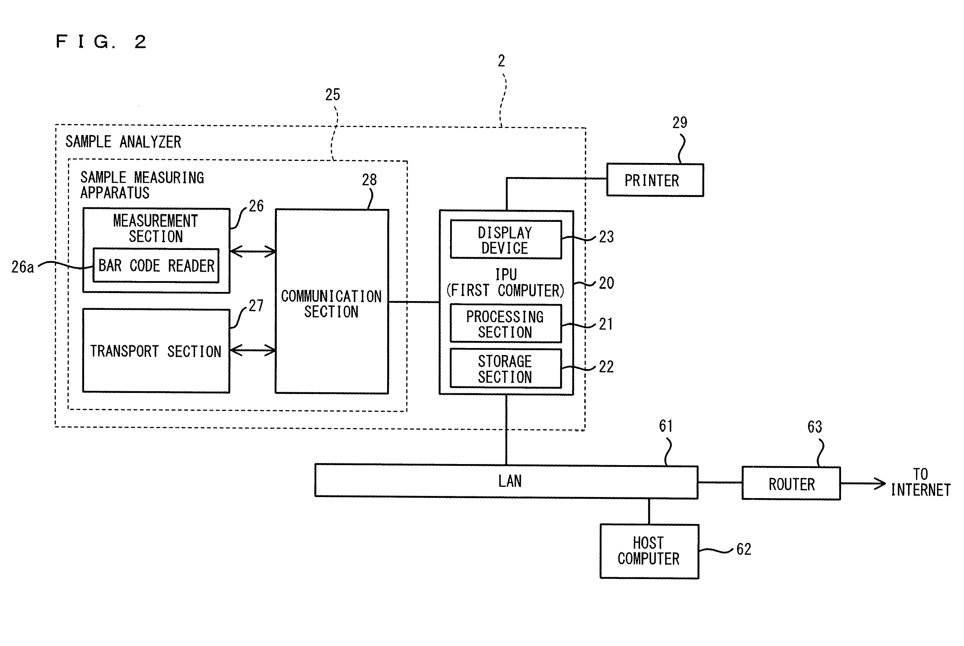

FIG. 2 shows a configuration of the sample analyzer 2 and apparatuses in the facility 200 relating to the sample analyzer 2 (a configuration of a network (LAN) in the facility 200). The sample analyzer 2 is, for example, a blood analyzer which analyzes, as samples, clinical samples (for example, blood) collected from patients. The sample analyzer 2 includes an information processing unit (IPU) 20, and a sample measuring apparatus (also referred to as body) 25. The information processing unit (first computer) 20 performs processing such as control of sample measurement performed in the sample measuring apparatus 25 and analysis of measurement data, and the like. The information processing unit 20 includes a processing section 21, a storage section 22, a display device 23, and the like, and has a function similar to that of a personal computer.

The storage section 22 is configured to include a hard disk and the like. The storage section 22 has stored (installed) therein computer programs for performing processing (processing such as control of sample measurement, analysis of measurement data, and the like) that needs to be performed in the processing section 21 of the information processing unit 20. Moreover, in the storage section 22, data (set values) necessary for performing the above processing, processing results, and the like are also stored. Contents of the storage section 22 will be described later. The processing section 21 includes a CPU, a ROM, a RAM, and the like, and can read out programs stored in the storage section 22 and execute them. The display device 23 can output and display analysis results of samples and can display an operation screen and the like for the sample analyzer 2. It should be noted that analysis results can be printed out by a printer 29 connected to the information processing unit 20.

The sample measuring apparatus 25 is a blood cell counter which includes a measurement section 26, a transport section 27, and a communication section 28. The measurement section 26 measures samples such as blood or the like. The transport section 27 transports racks on which sample containers each containing a sample are placed, to the measurement section 26. The measurement section 26 includes an aspirating pipette, a sample preparation portion, and a detecting portion. The aspirating probe aspirates a sample in a sample container transported by the transport section 27. The sample preparation portion prepares a measurement sample by mixing the aspirated sample and a regent. The detecting portion performs a measurement (sample measurement processing) on the prepared measurement sample to count blood cells contained therein. To each sample container, a bar code label indicating a sample ID for identifying an individual sample is attached. The measurement section 26 includes a bar code reader (ID obtaining part) 26a which reads a bar code of each sample container placed on a rack, and by the bar code reader 26a reading the bar code of the sample container, the information processing unit 20 can recognize the sample ID. Detail description of the configuration of the sample measuring apparatus 25 can be found in the U.S. Application Publication No.2010-0330609 assigned to the applicant of the present application. Entire disclosure of U.S. Application Publication No.2010-0330609 is herein incorporated by reference. The blood cell counter is one example of a sample measuring apparatus. Needless to say, another type of sample measuring apparatus can be employed instead of the blood cell counter, for example, immunological analyzer, blood coagulation analyzer, urine analyzer, and so on.

The communication section 28 is in charge of communication between the measurement section 26 and the transport section 27, and the information processing unit 20. A command/data transmitted from the information processing unit 20 is provided to the measurement section 26 or the transport section 27 via the communication section 28, and information (such as sensor detection results and the like) obtained by the measurement section 26 and the transport section 27 is transmitted to the information processing unit 20 via the communication section 28.

The information processing unit 20 of the sample analyzer 2 is connected to a LAN 61 in the facility 200, and can communicate with a host computer 62 in the facility 200 via the LAN 61. Further, the information processing unit 20 can access the Internet 4 being an external network, via the LAN 61 and a router 63. It should be noted that the printer 29 described above may be a network printer connected to the LAN 61.

The host computer 62 is installed in the facility 200 and is a computer that comprehensively manage pieces of measurement order information to be issued to the plurality of sample analyzers 2 installed in the facility 200 and analysis results obtained by the sample analyzers 2 performing measurements and analyses of samples in accordance with the pieces of measurement order information. When a sample ID assigned to a sample is transmitted from the sample analyzer 2, the host computer 62 transmits measurement order information corresponding to that sample ID to the information processing unit 20 (real time inquiry). Measurement order information is information indicating items and the like to be measured for an individual sample represented by a sample ID. Measurement order information includes a sample ID, a patient ID of a patient from whom a sample has been collected, and items to be measured for the sample. It should be noted that the patient ID included in measurement order information is for making reference to a patient database managed by the host computer 62. As described later, although measurement order information, measurement data, and an analysis result are transferred to an external server to be stored therein, these include only the patient ID, and thus, information, such as a patient name, that allows identification of the patient is not transmitted to the external server.

Upon receiving measurement order information from the host computer 62, the information processing unit 20 stores that measurement order information in the storage section 22, and causes the measurement section 26 to perform measurement in accordance with the measurement order information. The measurement section 26 returns measurement data to the information processing unit 20. The information processing unit 20 stores the obtained measurement data in the storage section 22, and also reports (transmits) an analysis result to the host computer 62 for result-reporting in return for the measurement order information.

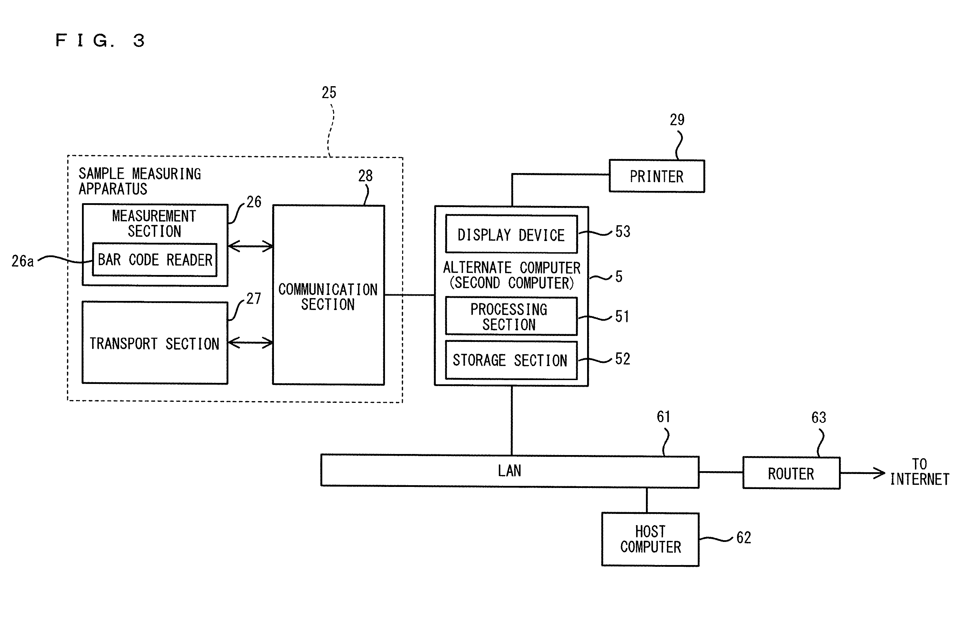

When the information processing unit 20 is normally operating, the sample analyzer 2 can perform by itself entire processing from causing the sample measuring apparatus 25 to measure a sample to performing analysis processing on measurement data obtained as a result of the measurement, independent of the external server 3. On the other hand, when the information processing unit 20 has failed, the information processing unit 20 shown in FIG. 2 is replaced with an alternate computer (second computer) 5 as shown in FIG. 3. That is, the alternate computer 5 is connected to the sample measuring apparatus 25 and the sample measuring apparatus 25 operates under control of the alternate computer 5.

As the alternate computer 5, a general commercially available personal computer can be used. That is, it is sufficient that the alternate computer 5 includes a processing section 51, a storage section 52, a display device 53, and the like, as in the case of the information processing unit 20.

The alternate computer 5 controls measurement on a sample in the sample measuring apparatus, as in the case of the information processing unit 20, but in the case of the alternate computer 5, unlike the information processing unit 20, the external server 3 takes charge of analysis processing which imposes a large processing load. That is, analysis processing is performed by cloud computing.

Here, when the information processing unit 20 has failed, it is conceivable that the alternate computer 5 performs all the functions of the information processing unit 20. However, in such a case, all software (computer programs) having been installed in the information processing unit 20 needs to be installed in the alternate computer 5, which takes time. When time taken for installing the programs is prolonged, resumption of operation of the sample analyzer is delayed accordingly, which causes a prolonged down time. In addition, in order for the alternate computer 5 to execute the software having been installed in the information processing unit 20, the alternate computer 5 is required to have a spec (free space of the hard disk, memory capacity, processing speed of the CPU (the processing section 51) equivalent to the spec of the information processing unit 20. It is often difficult to immediately prepare such a high spec computer.

The alternate computer 5 of the present embodiment performs only a part (control of sample measurement) of functions having been performed by the information processing unit 20, and thus, need not perform the other functions (analysis processing). Accordingly, less software is necessary for the alternate computer 5, and thus, installation of such software can be quickly performed. Moreover, since functions of software installed in the alternate computer 5 are limited, the alternate computer 5 may have a low spec. Thus, the user (the facility 200) of the sample analyzer 2 can easily prepare the alternate computer 5.

On the other hand, when the information processing unit 20 has failed, it is also conceivable that the external server 3 takes charge of not only analysis processing but also control of sample measurement performed by the sample measuring apparatus 25. However, some hardware control of the sample measuring apparatus 25 includes processing that requires response on the order of several tens to hundred milliseconds, and such a high-speed response is very difficult to be stably provided via the Internet 4. In this point, in the present embodiment, hardware control of the sample measuring apparatus 25 is performed by the alternate computer 5 directly connected to the sample measuring apparatus 25 via a USB or a LAN. Thus, high-speed response to the sample measuring apparatus 25 is allowed while cloud computing is being used for analysis processing.

### [2. Stored contents of information processing unit and external server]

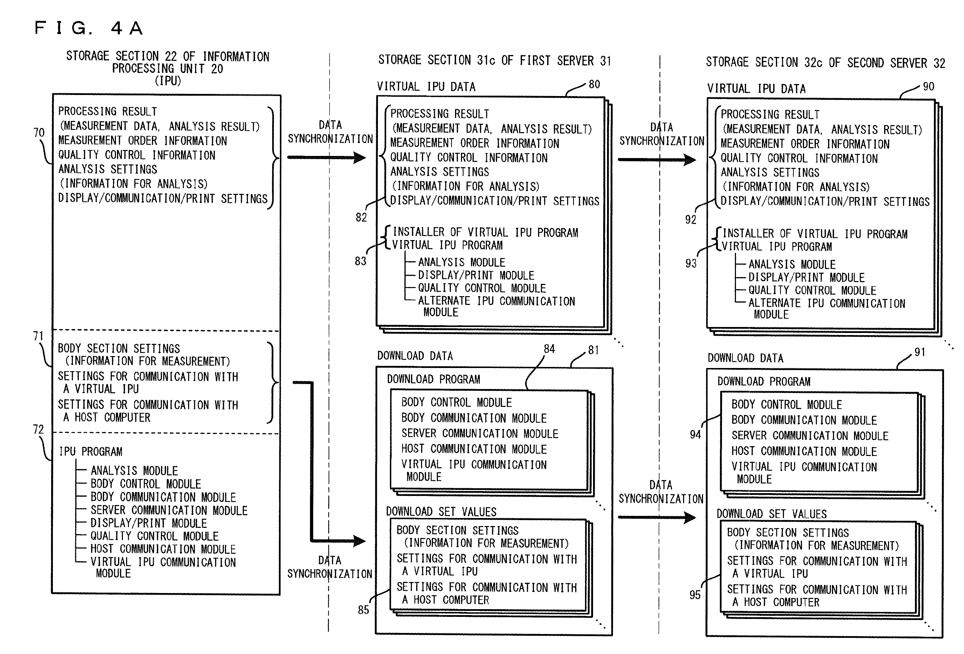

As shown in FIG. 4A, in the storage section 22 of the information processing unit 20, data 70 and 71 and a computer program (IPU program) 72 are stored. The IPU program 72 causes the information processing unit 20 to perform processing of controlling the sample measuring apparatus 25 (control of sample measurement performed in the measurement section 26 and control of the transport section 27) and analyzing measurement data of samples performed by the measurement section 26.

The IPU program 72 includes a plurality of modules separately configured in accordance with functions. Specifically, the IPU program 72 includes an analysis module, a body control module, a body communication module, a server communication module, a display/print module, a quality control module, a host communication module, a virtual IPU communication module, and the like. It should be noted that, in the present specification, a program unit that performs a defined function will be referred to as a module.

The analysis module is a program having a function of generating an analysis result, by analyzing measurement data generated by the measurement section 26, with reference to predetermined analysis condition settings.

The body control module is a program having a function of causing the body (the measurement section 26 and the transport section 27) to perform measurement of necessary measurement items in accordance with measurement order information. Specifically, the body control module creates operation schedules which schedule operation necessary in order for operating parts of the body (the measurement section 26 and the transport section 27) to measure measurement items. For creation of the operation schedules, predetermined body section settings are referred to. The created operation schedules are allocated to the respective operating parts by driver circuits of the body (the measurement section 26 and the transport section 27).

The body communication module is a program having a function of transmitting the operation schedules created by the body control module to the body (the measurement section 26 and the transport section 27), and a function of receiving, from the measurement section 26, measurement data generated by the measurement section 26, sample IDs obtained from the bar codes of sample containers, and the like.

The server communication module is a program having a function of performing communication with the external server 3. The server communication module allows transmission, to the external server in real time, of difference, i.e., change, having occurred in data such as measurement data, analysis results, various set values, and the like in the storage section 22.

The display/print module is a program having a function of causing the display device 23 to display data, and of outputting print data to the printer 29, and the like.

The quality control module is a program having a function of creating a quality control chart based on an analysis result of a quality control sample.

The host communication module is a program having a function of performing communication with the host computer 62. The host communication module allows transmission of an inquiry command for measurement order information to the host computer 62, and reception of measurement order information from the host computer 62.

The virtual IPU communication module is a program to be executed by the alternate computer 5 and having a function of performing communication with the virtual IPU 31b constructed in the external server 3. The virtual IPU communication module is a module for the alternate computer 5 and is not directly necessary for the information processing unit 20.

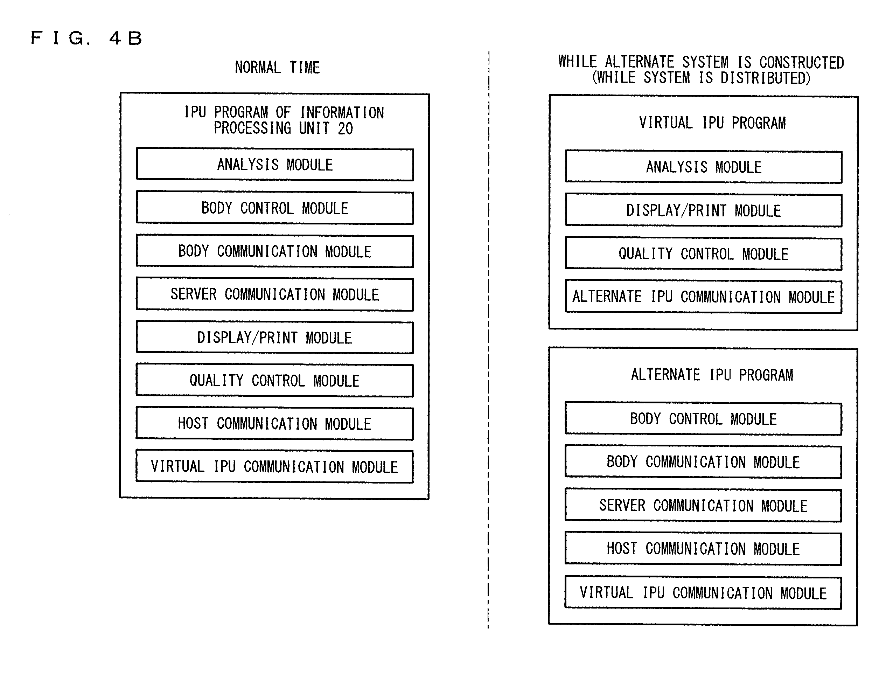

FIG. 4B is a schematic diagram showing how modules are allocated during normal time and while an alternate system is constructed (while the system is distributed). As shown in FIG. 4B, during normal time, by the information processing unit 20 executing the IPU program, the information processing unit 20 can execute all the modules. That is, the information processing unit 20 can perform a series of processes of measurement of samples, generation of analysis results, and reporting to the host computer 62, independently of the external server 3. On the other hand, while the system is distributed, among the modules in the IPU program, the analysis module, the display/print module, and the quality control module are allocated to the virtual IPU 31b. On the other hand, the body control module, the body communication module, the server communication module, the host communication module, and the virtual IPU communication module are allocated to the alternate computer 5. That is, the alternate computer 5 holds only functions of controlling hardware of the body (the measurement section 26 and the transport section 27) and functions of performing relay among the body, the external server 3, and the host computer 62, and functions such as analysis and the like are all to be performed by the virtual IPU.

With reference back to FIG. 4A, the data 70 and 71 stored in the storage section 22 are data relating to sample measurement or analysis of measurement data. The data 70 and the data 71 include first data 70 and second data 71. The data 70 and 71 are data unique to an individual sample analyzer 2, and set values contained in the data 70 and 71 are system information that determines operation of the sample analyzer 2. If the set values unique to the sample analyzer 2 are lost, the user (the facility 200) can no longer cause the sample analyzer 2 to operate under the same condition as before.

The first data 70 is information to be accessed by the virtual IPU 31b when the alternate computer 5 is used instead of the information processing unit 20. The first data 70 includes processing results (measurement data, analysis results), measurement order information, quality control information, analysis settings (information for analysis), and display/communication/print settings.

Each processing result is data representing a processing result obtained in the sample analyzer 2. The processing result includes measurement data obtained by the sample measuring apparatus 25 (raw data outputted from the measurement section 26), and an analysis result obtained by the information processing unit 20 analyzing the measurement data. The measurement order information is information representing items and the like to be measured with respect to a sample, as described above. The information processing unit 20 receives measurement order information from the host computer 62 and stores the measurement order information in the storage section 22.

The quality control information is information regarding quality control. The quality control information includes quality control set values which are set regarding operation of the measurement section 26 for analysis quality control and analysis processing for quality control, and analysis results obtained through analysis of measurement data which has been obtained by the measurement section 26 measuring a quality control substance (quality control sample).

The analysis settings (information for analysis) are set values used when measurement data of a sample obtained by the measurement section 26 is to be analyzed. The analysis settings include, for example, calibration curve information, flagging conditions, calibration values. The calibration curve information is information defining the relationship between measurement values and concentrations, the relationship being used for converting measurement data (raw data) outputted from the measurement section 26 into a concentration. The flagging conditions are conditions each for setting, in a case where a value of an analysis result is outside a range of numerical values indicating normal values, a flag indicating that the analysis result is an abnormal value, at the time of outputting the analysis result. The calibration values are values each indicating a correction factor used when measurement data (raw data) is converted into an analysis result. The display/communication/print settings include set values regarding display/communication/print.

The second data 71 is information downloaded by the alternate computer 5 and accessed (referred to) by the alternate computer 5, when the alternate computer 5 is used instead of the information processing unit 20. The second data 71 includes body section settings (information for measurement), settings for communication with a virtual IPU, and settings for communication with a host computer. The body section settings (information for measurement) are set values regarding the sample measuring apparatus 25. The body section settings include, for example, a serial ID of the sample measuring apparatus 25 (the sample analyzer 2), an IP address of the sample measuring apparatus 25, information indicating presence/absence of options, measurement suspension conditions, sensitivity adjustment values, position adjustment data for measurement components of the sample measuring apparatus 25, and the like. The serial ID is an ID unique to an individual sample measuring apparatus 25. The serial ID also includes information specifying the model of the sample measuring apparatus 25 (the sample analyzer 2), and thus, by use of the serial ID, the model of the sample measuring apparatus 25 (the sample analyzer 2) can also be specified.

The information indicating presence/absence of options is information indicating whether options are added to the sample measuring apparatus 25. Each measurement suspension condition shows a condition based on which measurement is suspended when abnormality has occurred in the measurement section 26 or the transport section 27. The user has selected, from a plurality of abnormality factors, abnormality factors based on which measurement should be suspended at an occurrence of abnormality, and the selected abnormality factors become the measurement suspension conditions. The abnormality factors are, for example, sample ID reading abnormality, sample aspiration abnormality, expiration of reagent use period, and the like.

The settings for communication with a virtual IPU are set values for performing communication with the virtual IPU of the first server 31. The settings for communication with a virtual IPU are set values for the alternate computer 5 and are information that is not directly necessary for the information processing unit 20. The settings for communication with a virtual IPU include an IP address provided to the virtual IPU.

The settings for communication with a host computer are set values for performing communication with the host computer 62. The settings for communication with the host computer 62 include an IP address of the host computer 62.

The IPU program 72 of the information processing unit 20, when executed, refers to the measurement order information, the analysis settings, and the display/communication/print settings included in the first data 70 and the body section settings included in the second data 71, and causes the sample measuring apparatus 25 to perform measurement of a sample in accordance with the measurement order. Moreover, the IPU program 72 stores, in the storage section 22, measurement data obtained from the measurement section 26, and an analysis result of the measurement data as a processing result. Moreover, at the time of quality control, the IPU program 72 performs processing for referring to the quality control set values and performing quality control, and stores, in the storage section 22, a result of the processing for the quality control as measurement data of the quality control.

As shown in FIG. 4, when the sample measuring apparatus 25 is capable of operating under control of the information processing unit 20, the information processing unit 20 performs data synchronization of the first data 70 and the second data 71 with the external server 3. Accordingly, replicas (copies) of the first data 70 and the second data 71 are created in the external server 3.

In the storage section 31 c of the first server 31 of the external server 3, a storage region for virtual IPU data 80 and a storage region for download data 81 are provided. The storage region for the download data 81 includes a storage region for download programs 84 and a storage region for download set values 85. The storage region for the virtual IPU data 80 and the storage region for the download set values 85 are provided, in association with each of the plurality of sample analyzers 2, 2. A data group composed of the pieces of the data 80 and 85 respectively corresponding to the plurality of sample analyzers 2, 2 can be searched, by use of a serial ID (data identification information) of each of the sample analyzers 2, 2 as a key. The download programs 84 are provided so as to respectively correspond to the models (types) of various sample analyzers 2. The program group (control program group) composed of the download programs 84 respectively corresponding to the models of the various sample analyzers 2 can be searched, by use of a model (program identification information) specified by a serial ID of each of the sample analyzers 2, 2 as a key.

The virtual IPU data 80 includes a first data replica 82 and an installer 83 for a virtual IPU program. The first data replica 82 is a replica of the first data 70 generated in the first server 31 through data synchronization (replication) with the information processing unit 20.

The virtual IPU program is a program for causing the external server 3 to function as the virtual IPU 31b. The virtual IPU program includes an analysis module, a display/print module, a quality control module, and an alternate IPU communication module. Among the modules included in the virtual IPU program, the analysis module, the display/print module, and the quality control module are modules having functions equivalent to the analysis module, the display/print module and the quality control module included in the IPU program 72. That is, the virtual IPU program is a program to be executed by the virtual IPU 31b in order to realize, among the functions (modules) of the IPU program 72 installed in the information processing unit 20, the analysis module (analysis program) for sample measurement data in the first server 31. When the virtual IPU 31b has become necessary (when the information processing unit 20 has failed and the alternate computer 5 is to be used), the virtual IPU program is automatically installed in the first server 31 by the installer 83, whereby the virtual IPU 31b is activated. The virtual IPU 31b performs sample analysis processing while cooperating with the alternate computer 5. It should be noted that, in a case where there are a plurality of the sample analyzers 2 that need the virtual IPU 31b, a plurality of the virtual IPUs 31b can be simultaneously activated in the first server 31.

The download program 84 is a program to be installed in the alternate computer 5 that is used when the information processing unit 20 has failed. The download program 84 includes a body control module (control program), a body section (the sample measuring apparatus 25) communication module, a server communication module, a host communication module, and a virtual IPU communication module. The respective modules included in the download program 84 are programs having functions equivalent to those of the modules that have names identical thereto in the IPU program. That is, the body control module is a program for causing the alternate computer 5 to perform processing of controlling sample measurement performed in the sample measuring apparatus 25 (the measurement section 26 and the transport section 27) being a measurement section. The body section communication module is a program for causing the alternate computer 5 to perform processing of performing communication with the sample measuring apparatus 25 being a measurement section. The server communication module is a program having a function of performing communication with the external server 3. The host communication module is a program having a function of performing communication with the host computer 62. The virtual IPU communication module is a program for causing the alternate computer 5 to perform processing of performing communication with the virtual IPU 31b.

The download program 84 is a minimum program necessary for the alternate computer 5 to control the sample measuring apparatus 25 while cooperating with the virtual IPU 31b. By executing the download program 84, the alternate computer 5 can realize a flow of receiving an instruction to measure a sample based on a measurement order from the virtual IPU 31b, causing the sample measuring apparatus 25 to perform measurement of the sample, receiving measurement data (raw data) from the sample measuring apparatus 25, and transferring the received measurement data (raw data) to the virtual IPU 31b. It should be noted that the download program 84 does not include a program for analysis processing performed by the virtual IPU 31b.

The download set values 85 are a replica (second data replica) of the second data 71 generated in the first server 31 through data synchronization (replication) with the information processing unit 20.

In the storage section 32c of the second server 32, a replica (virtual IPU data replica) 90 of the virtual IPU data 80 of the first server 31, and a replica (download data replica) 91 of the download data 81 of the first server 31 are generated through data synchronization (replication) with the first server 31.

### [3. Method for implementing sample analysis system]

### [3.1 Sample analysis method during normal time (during normal operation of information processing unit)]

In a case where the information processing unit 20 can normally operate (when not having failed), the sample measuring apparatus 25 can operate under control of the information processing unit 20 connected to the sample measuring apparatus 25 (see FIG. 2). In this case, when a difference (change) has occurred in the first data 70 or the second data 71 of the information processing unit 20, the difference data (content of change) is transmitted to the first server 31, and the virtual IPU data 80 or the download set values 85 of the first server 31 is overwritten (replication) with the difference data. Change in the first data 70 or the second data 71 occurs when a set value has been changed by the user or when measurement data or an analysis result has been generated.

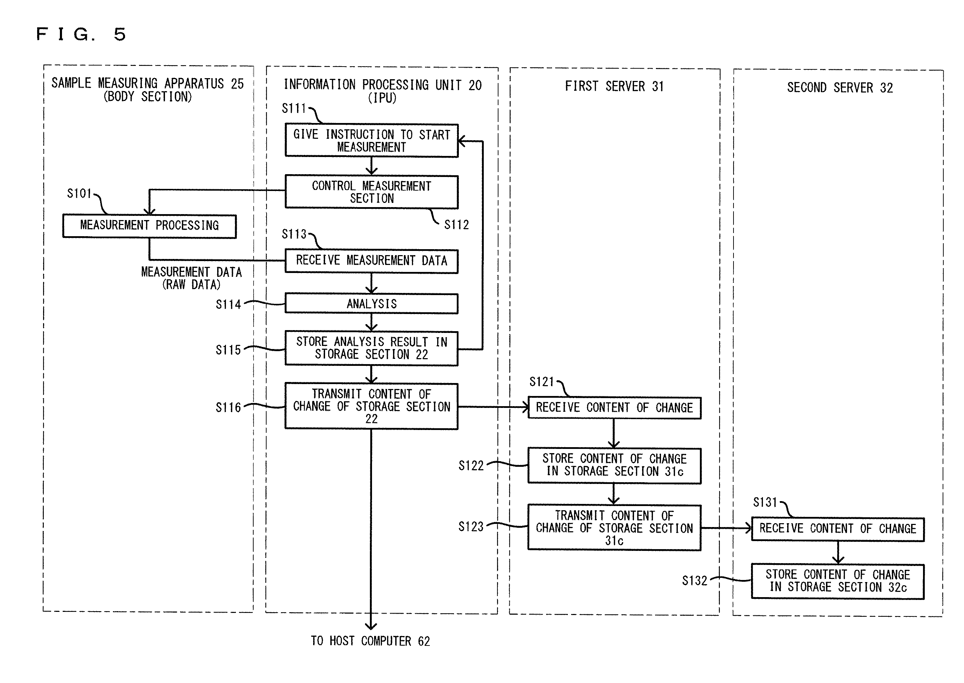

FIG. 5 shows a procedure of data synchronization (replication) of an analysis result obtained through measurement processing and analysis processing. First, upon receiving a measurement start instruction from the user via an input section such as a keyboard, a mouse, a touch panel, or the like provided to the information processing unit 20 (step S111), the information processing unit 20 transmits a command to start measurement to the sample measuring apparatus 25 (step S112). It should be noted that the command to start measurement may be issued via operation of a button provided in the sample measuring apparatus 25.

The sample measuring apparatus 25 under control of the information processing unit 20 performs measurement processing upon receiving the command to start measurement (step S101). Specifically, first, the transport section 27 transports a sample container to the measurement section 26. The bar code reader 26a of the measurement section 26 reads the bar code of the transported sample container. The result of reading the bar code (sample ID) is transmitted to the information processing unit 20 via the communication section 28. The information processing unit 20 makes an inquiry about measurement order information to the host computer 62, using the sample ID as a key. The host computer 62 searches for measurement order information corresponding to the sample ID and transmits the obtained measurement order information to the information processing unit 20. The information processing unit 20 stores the received measurement order information in the storage section 22. The information processing unit 20 transmits, to the measurement section 26 (the sample measuring apparatus 25), a measurement instruction for causing the measurement section 26 to perform measurement in accordance with the measurement order information. Specifically, the information processing unit 20 creates operation schedules for mechanism parts of the measurement section 26 and the transport section 27 such that measurement in accordance with the measurement order information can be performed, and transmits the created operation schedules to the measurement section 26 and the transport section 27. The transport section 27 transports a sample rack in accordance with the operation schedule, and the measurement section 26 performs measurement of necessary measurement items in accordance with the operation schedule, and outputs measurement data (raw data). When controlling the measurement section (step S112), the information processing unit 20 refers to the body section settings (information for measurement) as necessary, to perform the control.

The information processing unit 20 receives, via the communication section 28, the measurement data outputted from the measurement section 26, and stores the measurement data in the storage section 22 (step S113). Subsequently, the information processing unit 20 obtains an analysis result by analyzing the measurement data (step S114). When analyzing the measurement data, the information processing unit 20 refers to the analysis settings (information for analysis) as necessary, to perform the analysis. The information processing unit 20 stores the obtained analysis result in the storage section 22 (step S115). The analysis result is outputted to the display device 23 and the like of the information processing unit 20. Further, the information processing unit 20 transmits the obtained analysis result to the host computer 62.

For performing data synchronization (replication) with the first server 31, the information processing unit 20 can detect differences in the first data 70 and the second data 71 in the storage section 22. When the analysis result (a part of the first data 70) is stored, the information processing unit 20 detects change in the first data 70, and transmits the content of the change (i.e., the stored analysis result) to the first server 31 (step S116).

Upon receiving the content of the change (analysis result) (step S121), the first server 31 stores the content of the change in a storage region for analysis results in the virtual IPU data 80 (step S122). Accordingly, the contents of the first data replica 82 of the first server 31 match those of the first data 70 of the information processing unit 20, whereby data synchronization is established.

For performing data synchronization (replication) with the second server 32, the first server 31 can detect changes in the virtual IPU data 80 and the download data 81 in the storage section 31c. When the received content of the change has been stored, the first server 31 detects change in the virtual IPU data 80, and transmits the content of the change to the second server 32 (step S123).

Upon receiving the content of the change (analysis result) (step S131), the second server 32 stores the content of the change in a storage region for analysis results in the virtual IPU data replica 90 (step S132). Accordingly, the contents of the virtual IPU data replica 90 of the second server 32 match those of the virtual IPU data 80 of the first server 31, whereby data synchronization is established.

The data synchronization between the information processing unit 20 and the first server 31 and the data synchronization between the first server 31 and the second server 32 as described above are also performed when contents other than the analysis results in the first data 70 and the second data 71 have been changed. Moreover, transmission of the content of change in data for data synchronization may be simultaneously performed (mirroring) to both of the first server 31 and the second server 32 from the information processing unit 20. In this case, the first server 31 need not transfer the content of the change to the second server 32.

### [3.2 During failure of information processing unit (alternate system construction)]

When the information processing unit 20 has become unavailable due to failure or the like, the alternate computer 5, instead of the information processing unit 20, is connected to the sample measuring apparatus 25 as shown in FIG. 3. The alternate computer 5 is also connected to the LAN 61, and thus, can access the external server 3 via the Internet 4, as in the case of the information processing unit 20. Here, it is assumed that, in the computer serving as the alternate computer 5, in its initial state of being connected to the LAN 61, only an operating system and application programs irrelevant to sample analysis are installed but programs relevant to sample measurement and sample analysis are not installed (i.e., in a state where a commercially available personal computer is connected as the alternate computer 5).

Hereinafter, description will be given of a procedure for constructing an alternate system (see FIG. 3) in which the alternate computer (alternate IPU) 5 is used instead of the failed information processing unit 20, to perform sample analysis in cooperation with the external server 3.

The alternate computer 5 does not analyze sample measurement data, but controls the sample measuring apparatus 25 in cooperation with the virtual IPU 31b. Therefore, it is necessary to install, in the alternate computer 5, programs necessary for the alternate computer 5 to control the sample measuring apparatus 25 in cooperation with the virtual IPU 31b. Moreover, in order for alternate computer 5 to control the sample measuring apparatus 25 while using the same set values as those of the failed information processing unit 20, it is necessary to set, in the alternate computer 5, the body section settings (information for measurement) set in the failed information processing unit 20. Further, the settings for communication with the virtual IPU 31b also need to be set in the alternate computer 5.

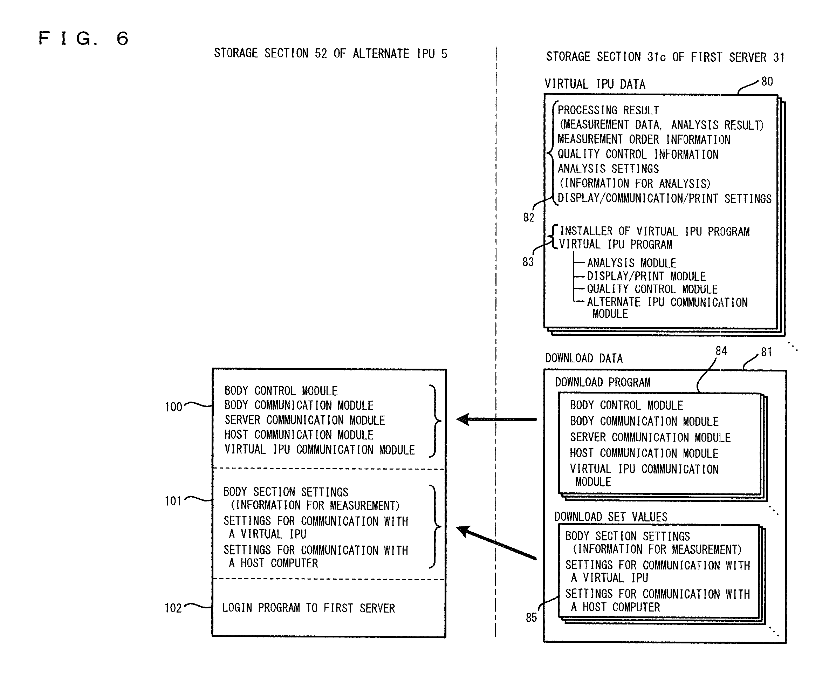

Programs and data (set values) necessary for the alternate computer 5 to control the sample measuring apparatus 25 in cooperation with the virtual IPU 31b exist as the download program 84 and the download set values 85 of the download data 81 in the first server 31. Thus, as shown in FIG. 6, it is sufficient that the alternate computer 5 downloads the download program 84 and the download set values 85 of the first server 31 and stores copies 100 and 101 thereof in the storage section 52. By the download data 81 being installed in the alternate computer 5 and the installed program being activated, the alternate computer 5 becomes able to control the sample measuring apparatus 25 in cooperation with the virtual IPU 31b. That is, the sample measuring apparatus 25 starts to operate under control of the alternate computer 5. It should be noted that the alternate computer 5 may be connected to the sample measuring apparatus 25 before the installation of the download data 81 or after the installation thereof.

Here, the alternate computer 5 needs a login program 102 for logging in the first server 31. However, in the present embodiment, as the login program 102, a function (remote login function, or the like) included in the operating system installed in the alternate computer 5 is used. Thus, the login program 102 need not be downloaded from the first server 31. However, it may be configured such that the login program 102 is downloaded from the first server 31 as necessary.

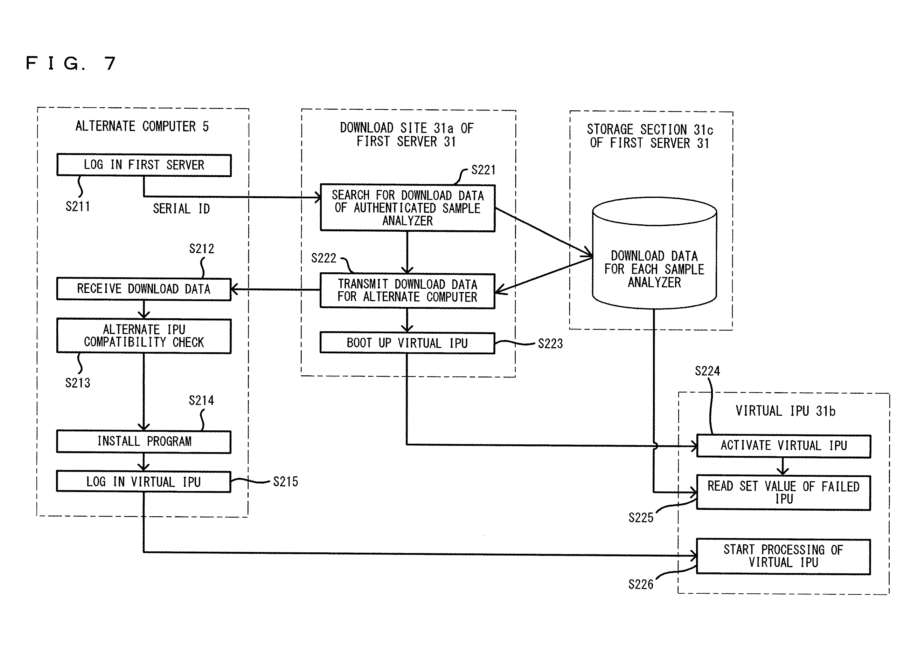

FIG. 7 shows a detailed procedure for constructing the alternate system. First, the user uses the login program 102 of the alternate computer 5 to log in the download site 31a of the first server 31 (step S211). At the time of login, the serial ID of the sample analyzer 2 is used as a login ID. After performing login authentication, the download site 3 1 a of the first server 31 searches for download data 81, using the serial ID as a key (step S221).

The serial ID of the sample analyzer 2 also includes information specifying the model of the sample analyzer 2. Therefore, when the program group of the download programs 84 is searched by use of the model (program identification information) specified by the serial ID as a key, a download program 84 corresponding to the model of the sample analyzer 2 can be extracted. Moreover, since the serial ID is information identifying an individual sample analyzer 2, when the data group of the download set values 85 is searched by use of the serial ID (data identification information) as a key, the set values (the body section settings and the settings for communication with a virtual IPU) transmitted from the failed information processing unit 20 can also be extracted.

The first server 31 transmits, to the alternate computer 5, the download data 81 (the download program 84 and the download set values 85) extracted through the search using the serial ID (step S222). Since the download data 81 is minimum data for performing control of the sample measuring apparatus 25 and thus, the data (program) size thereof is small, the download data 81 can be quickly downloaded.

Moreover, the first server 31 searches the data group of the virtual IPU data 80, using the serial ID (data identification information) as a key, and extracts a virtual IPU data 80 including the replica 82 of the first data 70 transmitted from the failed information processing unit 20. The first server 31 executes the installer 83 of the extracted virtual IPU data 80, to boot up the virtual IPU 31b in the first server 31 (step S223).

The alternate computer 5 obtains (downloads) the download data 81 (including the control program and the body section settings (information for measurement)) transmitted from the first server 31 (step S212). The alternate computer 5 stores, in the storage section 52, the received download data 81 as the copies 100 and 101 of the download data 81 of the first server 31, and starts installing the download program (body control module and the like). Prior to the actual installation, compatibility (memory capacity, version of the operating system, and the like) of the alternate computer 5 is checked (step S213). If there is no problem with the compatibility, the download program (body control module and the like) is installed (step S214). As a result, the alternate computer 5 becomes able to control the sample measuring apparatus 25 in the same environment as that of the failed information processing unit 20, in cooperation with the virtual IPU 31b.

Upon the activation of the virtual IPU 31b in the first server 31 (step S224), the virtual IPU 31b reads the first data replica (the set values/processing results and the like of the failed information processing unit (IPU) 20) of the virtual IPU data 80 previously extracted (step S225). Accordingly, the virtual IPU 31b can start sample analysis processing in the same environment as that of the failed information processing unit 20 (step S226).

The user of the sample analyzer 2 (alternate system) logs in the virtual IPU 31b of the activated alternate computer 5 (step S215). This login may be automatically performed by the alternate computer 5. The login to the virtual IPU 31b is performed by a remote desktop function included in the operating system of the alternate computer 5.

Through the above alternate system construction processing, an alternate system can be obtained in which the functions of the failed information processing unit 20 are reproduced while being distributed between the alternate computer 5 and the virtual IPU 31b. By logging in the virtual IPU 31b, the alternate computer 5 can receive, without performing analysis processing of sample measurement data by itself, results of analysis processing through cloud computing performed by the virtual IPU 31b.

### [3.3 While alternate system is in operation]

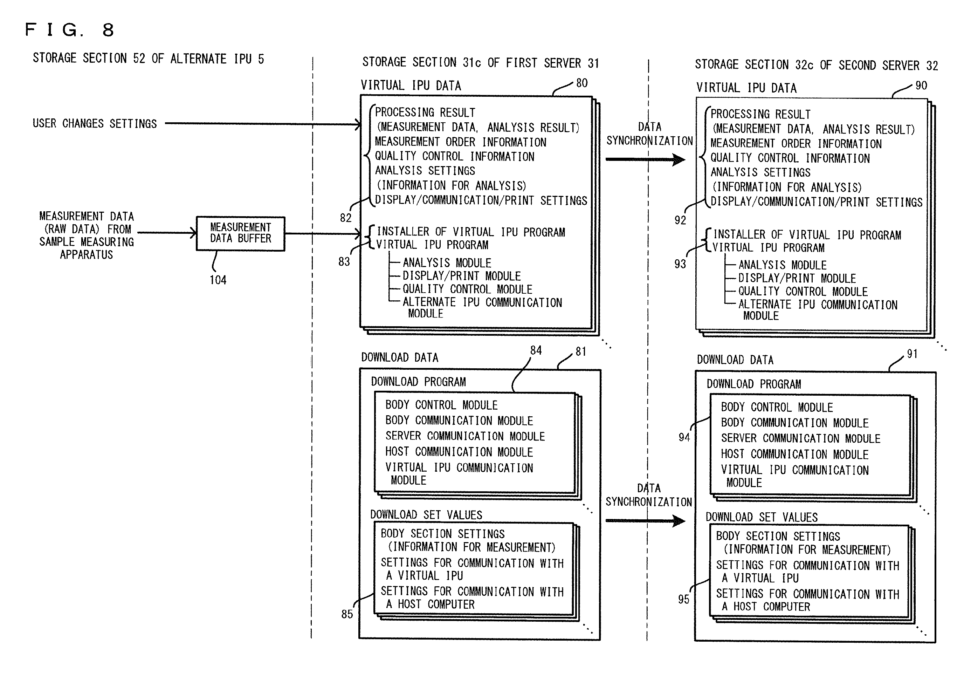

While the alternate system is in operation, the sample measuring apparatus 25 operates under control of the alternate computer (second computer) connected to the sample measuring apparatus 25 (see FIG. 3). During normal time, there are cases where some set values are changed or processing results are generated, whereby changes are made in the first data 70 and the second data 71 of the information processing unit 20. Similarly, also while the alternate system is in operation, there are cases where some set values are changed by the user or processing results (measurement data, analysis result) are generated, whereby changes are made in the contents of the storage section 31 c (the first data replica 82 and second data replica 85) of the first server 31 (see FIG. 8).

When change is made in the contents (the first data replica 82 and the second data replica 85) in the storage section 31c of the first server 31, the change is reflected on the second server 32 through data synchronization (replication) between the first server 31 and the second server 32.

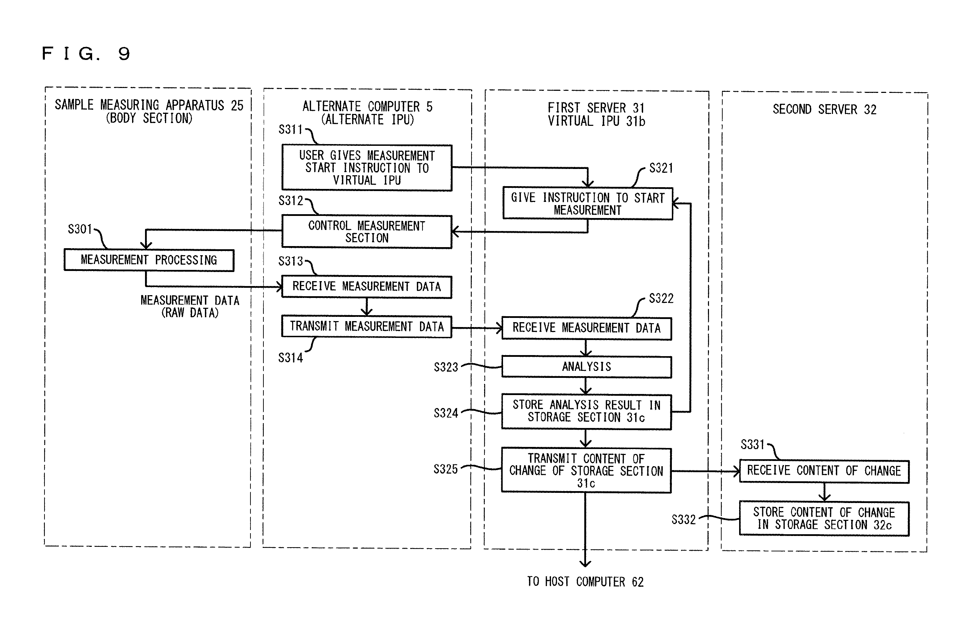

FIG. 9 shows a procedure of sample measurement processing, analysis processing, and data synchronization (replication) performed in the alternate system. First, the alternate computer 5 receives a measurement start instruction from the user via an input section such as a keyboard, a mouse, a touch panel, or the like provided to the alternate computer 5 (step S311). Since the alternate computer 5 has logged in the virtual IPU 31b, this measurement start instruction is a measurement start instruction addressed to the virtual IPU 31b. That is, since the user performs an operation of giving a measurement start instruction, from the operation screen displayed on the alternate computer 5, the measurement start instruction is apparently given to the alternate computer 5, but actually, the user remotely operates the operation screen generated by the virtual IPU. The virtual IPU 31b transmits instructions necessary for measurement, to the alternate computer 5 (step S321).

The alternate computer 5 receives a measurement instruction from the virtual IPU 31b by using virtual IPU communication module. Then, the body control module (control program) of the alternate computer 5 transmits commands necessary for measurement to the sample measuring apparatus 25, thereby controlling the sample measuring apparatus 25 (step S312). Control of the sample measuring apparatus 25 is performed by the alternate computer 5 in cooperation with the virtual IPU 31b.

The sample measuring apparatus 25 under control of the alternate computer 5 performs measurement processing (step S301). Specifically, first, the transport section 27 transports a sample container to the measurement section 26. The bar code reader 26a of the measurement section 26 reads the bar code of the transported sample container. The result of reading the bar code (sample ID) is transmitted to the alternate computer 5 via the communication section 28. The alternate computer 5 transfers the sample ID to the virtual IPU 31b. The virtual IPU 31b generates a command to inquire about measurement order information, using the sample ID as a key, and transmits the command to the alternate computer 5. The alternate computer 5 transfers the received command to the host computer 62. The host computer 62 searches for measurement order information corresponding to the sample ID and transmits the obtained measurement order information to the alternate computer 5. The alternate computer 5 transfers the received measurement order information to the virtual IPU 31b. The virtual IPU 31b stores the received measurement order information in the storage section 31c of the first server 31.

The virtual IPU 31b transmits, to the alternate computer 5, a measurement instruction for causing the measurement section 26 to perform measurement in accordance with the measurement order information stored in the storage section 31c. Based on the measurement instruction, the alternate computer 5 causes the measurement section 26 to perform measurement of the sample. Specifically, the alternate computer 5 creates operation schedules for mechanism parts of the measurement section 26 and the transport section 27 such that measurement in accordance with the measurement order information can be performed, and transmits the created operation schedules to the measurement section 26 and the transport section 27. The transport section 27 transports a sample rack in accordance with the operation schedule, and the measurement section 26 performs measurement of necessary measurement items in accordance with the operation schedule, and outputs measurement data (raw data). When controlling the measurement section (step S312), the alternate computer 5 refers to the body section settings (information for measurement) stored in the storage section 52 as necessary, to perform the control.

The alternate computer 5 receives, via the communication section 28, the measurement data outputted from the measurement section 26 (step S313). The alternate computer 5 accumulates the received measurement data in a measurement data buffer 104 as necessary (see FIG. 8), and transmits the measurement data to the virtual IPU 31b (step S314).

Upon receiving the measurement data, the virtual IPU 31b stores the measurement data in the storage section 31c (step S322). Subsequently, the virtual IPU 31b obtains an analysis result by analyzing the measurement data (step S323). That is, analysis is performed by the first server 31 executing the analysis program. When analyzing measurement data, the virtual IPU 31b refers to the analysis settings (information for analysis) included in the virtual IPU data 80 stored in the storage section 31 c as necessary, to perform the analysis.

The virtual IPU 31b stores the obtained analysis result in the storage section 31c (step S324). The alternate computer 5 can refer to the obtained analysis result, by using the remote desktop function. Further, in order to report the analysis result to the host computer 62, the virtual IPU 31b transmits the analysis result to the alternate computer 5, first. Specifically, the virtual IPU 31b sets the IP address of the transmission destination of the analysis result to the IP address of the alternate computer 5, and transmits the analysis result. By re-setting the IP address of the received analysis result to the IP address of the host computer 62, the alternate computer 5 transmits the analysis result to the host computer 62. That is, the alternate computer 5 plays a role like a router that transfers the analysis result from the virtual IPU 31b to the host computer.

It should be noted that the virtual IPU 31b may directly transmit the analysis result to the host computer 62, not via the alternate computer 5. In such a configuration, load on the alternate computer 5 associated with transfer of the analysis result can be further reduced. However, when such a configuration is employed, data is directly transmitted/received between the external server 3 and the host computer 62, and thus, higher security is required compared with the environment where data is transmitted/received via the alternate computer.

For performing data synchronization (replication) with the second server 32, the virtual IPU 31b of the first server 31 transmits, upon detecting change in the virtual IPU data occurring when the analysis result was stored, the content of the change (i.e., the stored analysis result) to the second server 32 (step S325).

Upon receiving the content of the change (analysis result) (step S331), the second server 32 stores the content of the change in the storage region for analysis results in the virtual IPU data replica 90 of the second server (step S332). Accordingly, the stored contents of the first server 31 match the stored contents of the second server 32, whereby data synchronization is secured.

The data synchronization between the first server 31 and the second server 32 as described above is also performed when contents other than the analysis results in the data 82 and 85 in the first server 31 have been changed.

### [3.4 Recovery]

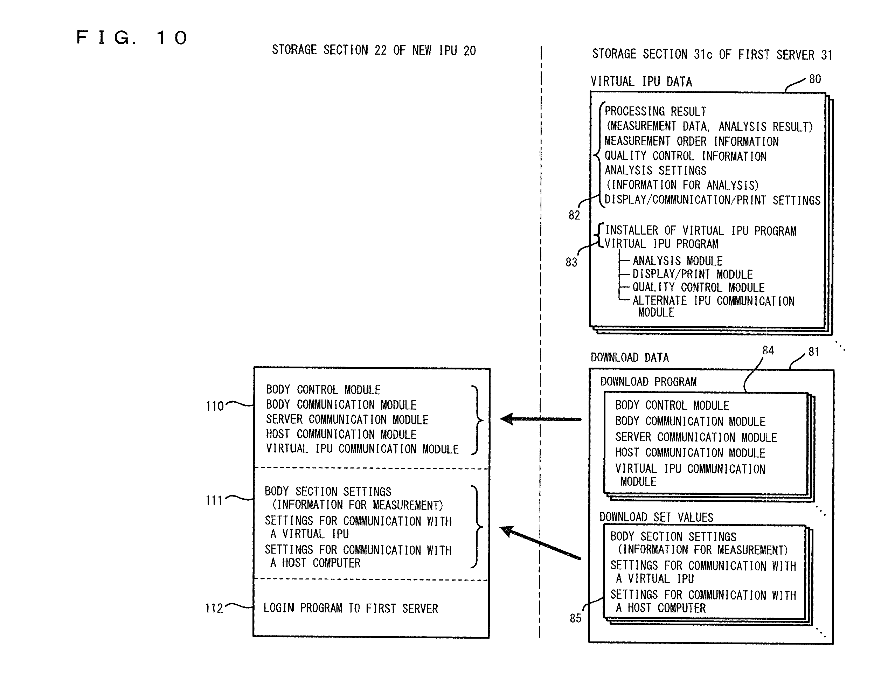

The alternate system need not be permanently used, and recovery to a state where analysis processing is performed by the information processing unit 20 connected to the sample measuring apparatus 25 (see FIG. 2) can be made. FIG. 10 to FIG. 12 show a procedure for the recovery.

As a recovery computer to be used in the recovery, the failed information processing unit 20 (first computer) which has been repaired may be used. Alternatively, a computer (third computer, new computer) having a hardware configuration and an operating system equivalent to those of the failed information processing unit 20 may be used. Still alternatively, the alternate computer 5 may be used.

In the following, description will be given of a case in which the failed information processing unit (first computer) 20 which has been repaired (new computer, new IPU) is used as a recovery computer to perform the recovery. It is assumed that, with respect to the new computer (new IPU) 20, the hard disk (the storage section 22) of the failed information processing unit 20 has been replaced or the like, and the IPU program 72 which had been installed in the information processing unit 20 before the information processing unit 20 failed is not installed therein. Moreover, all of the data 70 and 71 (set values, processing results, and the like) which had been stored in the information processing unit 20 before the information processing unit 20 failed are also lost.

First, prior to the recovery, a state where the new computer 20, instead of the alternate computer 5, is connected to the sample measuring apparatus 25 and the LAN 61 is made (see FIG. 2). Then, through the same procedure as the procedure taken in the alternate system construction, an alternate system using the new computer 20 is constructed. That is, as shown in FIG. 10, the new computer 20 downloads the download program 84 and the download set values 85 in the first server 31, and stores copies 110 and 111 thereof in the storage section 22. By the download data 81 being installed in the new computer 20 and the installed program being activated, an alternate system in which the new computer 20 and the virtual IPU 31b operate in cooperation with each other can be constructed. That is, the sample measuring apparatus 25 starts to operate under control of the new computer 20.

It should be noted that the new computer 20 may be connected to the sample measuring apparatus 25 before the installation of the download data 81 or after installation thereof. Further, a login program 112 shown in FIG. 10 is included in the operating system of the new computer 20.

By constructing the alternate system using the new computer 20, even after the alternate computer 5 has been replaced with the new computer 20 in association with the recovery operation, measurement by the sample measuring apparatus 25 and analysis processing by the virtual IPU 31b can be continued in an alternate system using the new computer 20 until the recovery operation is completed. In a case where the recovery is performed by using the alternate computer 5 as the new computer 20, since the alternate system is already constructed, alternate system construction for the recovery can be omitted.

Through the processing of constructing the alternate system using the new computer 20, as shown in FIG. 10, data (the body section settings (information for measurement), the settings for communication with a virtual IPU) 111 corresponding to the second data 71 (see FIG. 4) is already obtained. In order to completely recover the system to the state before the failure, i.e., to a state where the new computer 20 can perform measurement to analysis by itself, it is further necessary to cause the new computer 20 to obtain data corresponding to the first data 70 (see FIG. 4). The data corresponding to the first data 70 is stored as the first data replica 82, 92 in the external server 3 (the first server 31 and the second server), and thus, it is sufficient that the new computer 20 downloads (obtains) the first data replica 82, 92 from the external server 3.

However, when the first data replica 82, 92 is to be transferred to the new computer 20 while a test is being continued by the sample analyzer 2, an addition (update) of a new analysis result to be made to the first data replica 82, 92 and copying of the first data replica 82, 92 to the new computer 20 will simultaneously occur. This could result in inconsistency of data. In other words, while sample analysis processing is being performed, recovery involving data transfer is difficult to be performed.

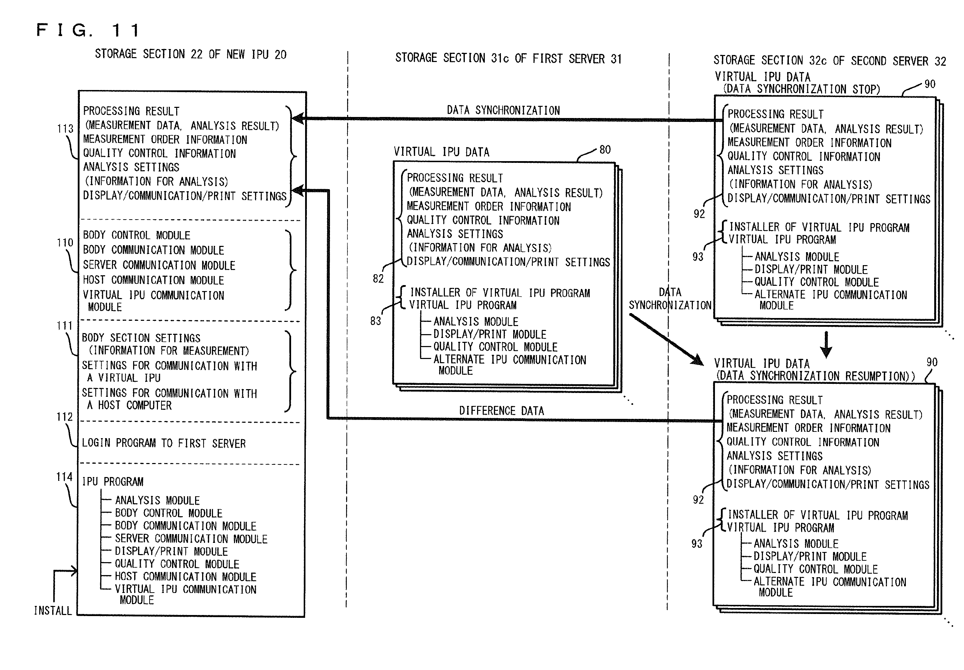

Thus, in the present embodiment, recovery is performed by using both of the first data replica 82 and 92, while sample analysis processing is continued without being suspended. That is, while the first server 31 maintains a state that allows data update (such as addition of analysis results), the second server 32 which has stopped data synchronization with the first server 31 transfers the first data replica 92 of the virtual IPU data 90, to the new computer 20, as shown in FIG. 11. Then, when update of data content is no longer performed in the first server 31 due to completion of necessary analysis or the like, the second server 32 resumes data synchronization with the first server 31, and obtains data (difference data) updated in the first server 31 while data synchronization was stopped. The difference data is provided to the new computer 20. Through the above procedure, the new computer 20 can obtain a copy 113 of the first data replica 92 in which data update made as a result of continuation of measurement and analysis is reflected.

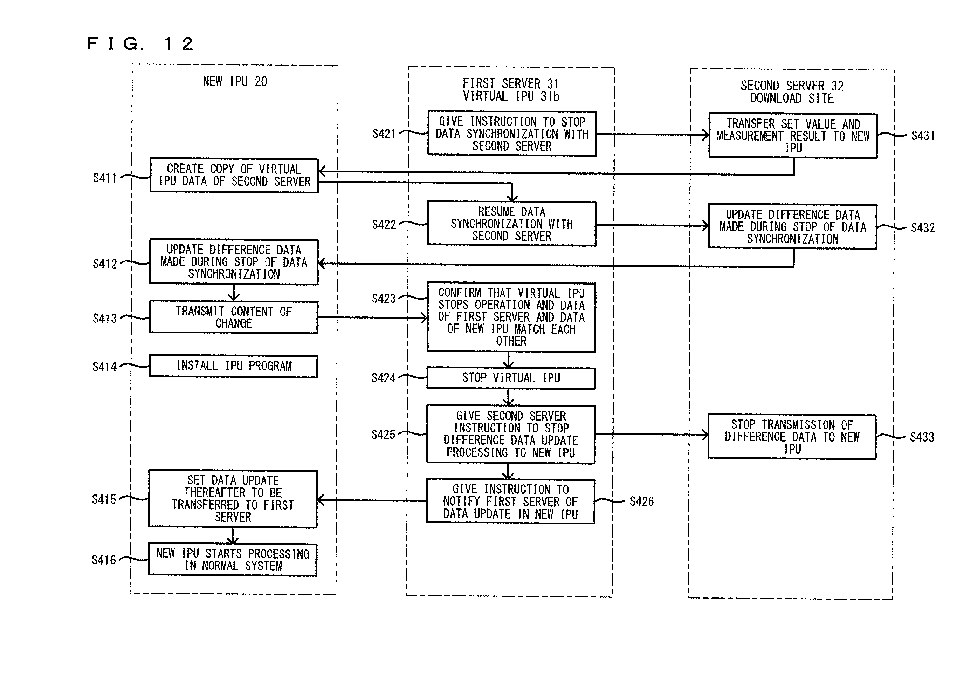

FIG. 12 shows a detailed procedure for the new computer 20 to obtain the first data replica 92 (the virtual IPU data 90). First, the virtual IPU 31b of the first server 31 transmits an instruction to stop data synchronization, to the second server 32 (step S421). Accordingly, the data synchronization between the first server 31 and the second server 32 stops. Upon stop of the data synchronization, the download site 32a of the second server 32 transfers, to the new computer 20, the first data replica 92 (such as set values, measurement data) of the virtual IPU data 90 stored in the storage section 32c (step S431).

While controlling the sample measuring apparatus 25, the new computer 20 receives the first data replica 92 of the virtual IPU data 90, and stores the copy 113 thereof in the storage section 22 (step S411). During transfer of the first data replica 92, measurement processing and analysis processing under cooperation between the new computer 20 and the virtual IPU 31b can be continued. Thus, the first data replica 82 (especially processing results) in the first server 31 may be updated even during transfer of the first data replica 92.

Upon completion of reception of the first data replica 92, the new computer 20 notifies the virtual IPU 31 b of the reception completion. After the virtual IPU 31 b has received the notification, when necessary measurement processing and necessary analysis processing have ended and no more data update will not occur, the virtual IPU 31b transmits an instruction to resume data synchronization, to the second server 32 (step S422). Accordingly, data synchronization between the first server 31 and the second server is resumed. That is, difference data that has occurred between the first server 31 and the second server 32 due to update made to the data of the first server 31 while data synchronization was stopped is transmitted to the second server 32, and the first data replica 92 of the second server 32 is updated, whereby the contents of the first data replica 92 match the contents of the first data replica 82 of the first server 31 (step S432).

Further, the difference data is transmitted from the download site 32a of the second server 32 to the new computer 20. The new computer 20 having received the difference data updates, with the difference data, the data 113 previously received (step S412). Further, the new computer 20 transmits the data update content (content of the change) made due to difference data, to the first server (step S413). Then, the first server 31 confirms, based on the received data update content (content of the change), that the data 113, 111 of the new computer 20 have matched the data 82, 85 of the first server 31 (step S423). When a state where such data synchronization has been realized has been obtained, the virtual IPU 31b stops (step S424). Moreover, the first server 31 gives, to the second server 32, an instruction to stop difference data update processing (data synchronization between the second server 32 and the new computer 20) (step S425). The second server having received the instruction stops transmission of difference data to the new computer 20 (step S433).

Through the above processing, a state where the data 113, 111 of the new computer 20, the data 82, 85 of the first server 31, and the data 92, 95 of the second server 32 have been all synchronized (the same state as FIG. 4, recovered state) is obtained. Further, when the state where the data synchronization has been realized has been obtained, an IPU program 114 (the same as the IPU program 72 shown in FIG. 4) is installed in the new computer 20. This installation may be performed before the above state of data synchronization is obtained. Accordingly, the new computer 20 becomes able to control the sample analyzer and to analyze sample measurement data.

Then, the first server 31 gives, to the new computer 20, an instruction to perform data synchronization (replication) associated with data update made in the new computer 20, with the first server 31, as in normal time (step S426). The new computer 20 having received the instruction is set to transfer data update thereafter to the first server 31. That is, it is set such that data synchronization is performed between the new computer 20 and the first server 31. Through the above procedure, the new computer 20 becomes able to function in the same manner as the information processing unit 20 did before the failure, and can perform operation during normal time as shown in FIG. 5 (step S416). It should be noted that, when the new computer 20 has started normal time operation, the modules 110 for causing the new computer 20 to operate in cooperation with the virtual IPU 31b are automatically or manually deleted.

### [4. Modification]

In the procedure of constructing the alternate system described above, when the download program (such as control program) 84 is installed in the alternate computer 5 (step S214 in FIG. 7) and the alternate system is constructed, sample measurement is directly enabled. In contrast, in modifications, sample measurement is performed after predetermined processing (operation confirmation of the alternate system, check of consistency of data with the host computer) is performed.

### [4.1 First modification (operation confirmation through execution of quality control)]

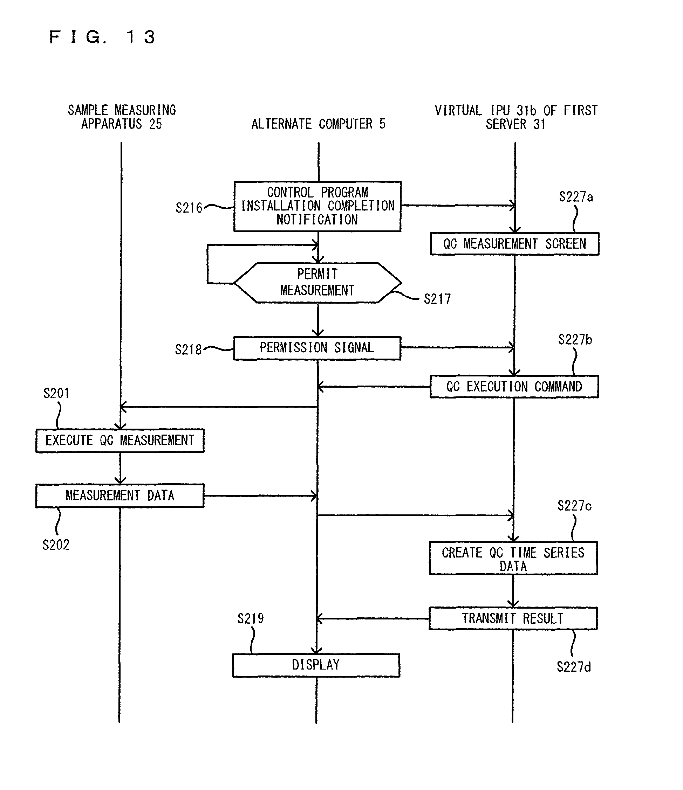

In a first modification, sample measurement is performed after operation confirmation of the alternate system is performed. First, when the download program (such as control program) 84 has been installed in the alternate computer 5 (step S214 in FIG. 7), an installation completion notification is transmitted from the alternate computer 5 to the virtual IPU 31b of the first server 31, as shown in FIG. 13 (step S216).

The virtual IPU 31b having received the installation completion notification generates a screen (QC measurement screen) that urges execution of quality control (QC) (step S227a). The generated QC measurement screen is transmitted to the alternate computer 5 logging in the virtual IPU 31b by the remote desktop function, and is displayed on the display device 53 of the alternate computer 5. The QC measurement screen includes, for example, a message that urges the user to set a quality control sample onto the sample analyzer 2, and an "OK" button display (measurement permission instruction button) which confirms that the quality control sample has been set.

When "OK" on the QC measurement screen has been selected (step S217), the alternate computer 5 transmits a permission signal for performing measurement, to the virtual IPU 31b (step S218). The virtual IPU 31b having received the permission signal transmits, via the alternate computer 5, a measurement execution command to measure a quality control sample, to the measurement section 26 of the sample measuring apparatus 25 (step S227 b). When generating the measurement execution command to measure a quality control sample, the virtual IPU 31b refers to the quality control information and the body section settings stored in the storage section 31 c, and generates the measurement execution command in accordance with the quality control information and the body section settings stored in the storage section 31 c.

The measurement section 26 performs measurement of a quality control sample in accordance with the measurement execution command (step S201), and transmits measurement data to the virtual IPU 31b via the alternate computer 5 (step S202). The virtual IPU 31b analyzes the received measurement data in accordance with the analysis settings stored in the storage section 31 c, to generate an analysis result. If the analysis result is an abnormal value, the virtual IPU 31b outputs an error message to be displayed on the alternate computer 5. Further, the virtual IPU 31b reads out past analysis results stored as quality control information in the storage section 3 1 c, and creates a time series data (QC time series data) on which the generated analysis result and past analysis results are plotted in time series (step S227c).

The virtual IPU 31b transmits, to the alternate computer 5, a quality control result screen including the QC time series data (in response to an operation from the alternate computer 5) (step S227d), and the alternate computer 5 displays the quality control result screen on the display device 53 (step S219). In a case where the generated analysis result is greatly different from the past analysis results in the QC time series data, it is known that analysis results differ before and after the failure of the information processing unit 20. In this case, there is a possibility that the constructed alternate system is inappropriate. Thus, the quality control result screen is made to include an error message or the like, thereby notifying the user the possibility of the alternate system being inappropriate.

### [4.2 Second modification (operation confirmation by sample measurement)]

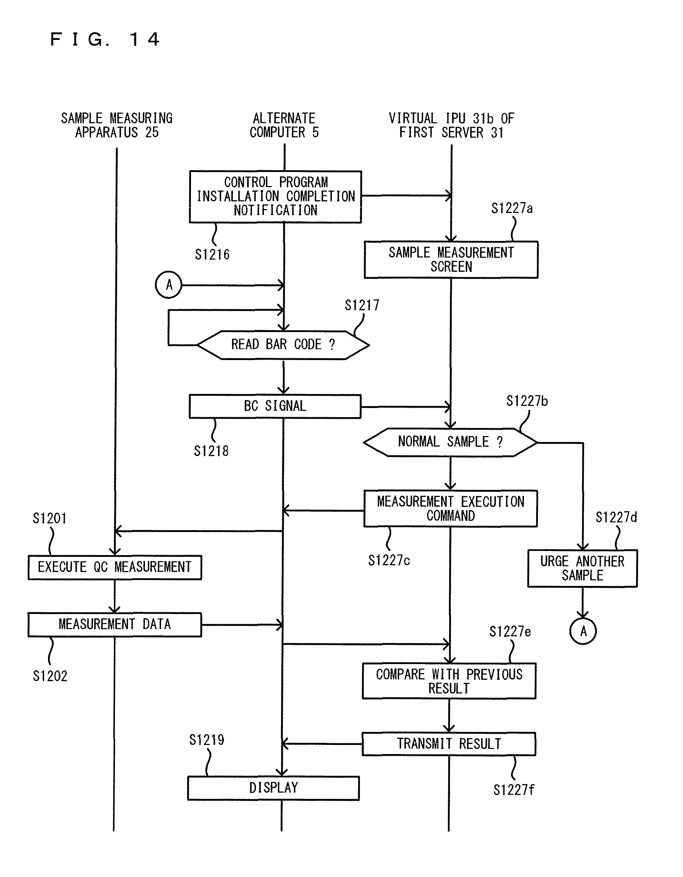

Operation confirmation of the alternate system can also be performed by remeasuring a sample that has been measured, instead of measuring a quality control sample. FIG. 14 shows a procedure of operation confirmation through re-measurement of a sample. When the download program (such as control program) 84 has been installed in the alternate computer 5 (step S214 in FIG. 7), installation completion notification is transmitted from the alternate computer 5 to the virtual IPU 31 b of the first server 31 (step S1216).

The virtual IPU 31b having received the installation completion notification generates a screen (sample measurement screen) which urges re-measurement of a sample that has been measured (step S1227a). The generated sample measurement screen is transmitted to the alternate computer 5 logging in the virtual IPU 31b by the remote desktop function, and displayed on the display device 53 of the alternate computer 5. The sample measurement screen includes, for example, a message that urges the user to cause the bar code reader 26a to read the bar code of a sample that has been measured. It should be noted that the bar code reader may be a handy bar code reader connected to the alternate computer 5, instead of the bar code reader 26 provided in the measurement section 26.

When the bar code of such a sample container has been read by the bar code reader 26a (step S1217), the alternate computer 5 transmits a result of reading the bar code (BC signal; sample ID) to the virtual IPU 31b (step S1218).

The virtual IPU 31b extracts a processing result (measurement data or analysis result) stored in the storage section 31 c, using the transmitted sample ID as a key, and determines whether the extracted processing result indicates a normal value (whether the sample indicated by the sample ID is normal) (step S1227b). When the sample is normal, in order to use that sample as a target for re-measurement, the virtual IPU 31b transmits a measurement execution command to the measurement section 26 of the sample measuring apparatus 25 via the alternate computer 5 (step S1227c). When the sample is abnormal, the sample is not appropriate for re-measurement for operation confirmation, and thus, the virtual IPU 31b causes the alternate computer 5 to display a screen including a message that urges the user to cause the bar code of another sample to be read (step S1227d).

The measurement section 26 having received the measurement execution command performs measurement of the sample (step S1201) and transmits measurement data thereof to the virtual IPU 31 b via the alternate computer 5 (step S1202).

The virtual IPU 31b analyzes the received measurement data to generate an analysis result. The generated analysis result is compared with the previously extracted past analysis result (step S1227e). The virtual IPU 31b transmits a result screen including a comparison result to the alternate computer 5 (in response to an operation from the alternate computer 5) (step S1227f), and the alternate computer 5 displays the result screen on the display device 53 (step S1219). When the difference between the generated analysis result and the past analysis result is within a predetermined range, the constructed alternate system is appropriate, and when the above difference is out of the predetermined range, there is a possibility that the constructed alternate system is inappropriate. Thus, the result screen is made to include an error message or the like, thereby notifying the user the possibility of the alternate system being inappropriate.

It should be noted that, during operation confirmation processing through quality control measurement, sample re-measurement, or the like, the virtual IPU 31b may obtain unregistered measurement order information from the host computer 62 via the alternate computer 5, and store the unregistered measurement order information in the storage section 31 c. Accordingly, the virtual IPU 31b can start measurement processing immediately after the operation confirmation, which is efficient.

### [4.3 Third modification (check of consistency of data with host computer)]

In a case where the information processing unit 20 failed and has been replaced with the alternate computer 5, discrepancy may occur between the transmission history/reception history of the alternate computer 5, and the reception history/transmission history of the host computer 62.

During normal time, in a case where real time inquiry about measurement order information is performed from the information processing unit 20 to the host computer 62, measurement order information is transmitted from the host computer 62 to the information processing unit 20. Then, the information processing unit 20 transmits an analysis result corresponding to the measurement order information to the host computer 62. In a case where these transmission and reception have been performed without any problem, correspondence between the transmission history/reception history of the information processing unit 20, and the reception history/transmission history of the host computer 62 is established.

However, in a case where the information processing unit 20 failed while receiving measurement order information from the host computer 62, correspondence between the transmission history and the reception history regarding the measurement order information may not be established. That is, even when the information processing unit 20 failed while receiving the measurement order information, the host computer 62 has a log of having transmitted the measurement order information inquired about. On the other hand, the information processing unit 20 which failed while receiving the measurement order information could not receive the measurement order information. Thus, the alternate computer 5 having downloaded data of the failed information processing unit 20 from the external server 3 has a log indicating not having received the measurement order information.

In a case where the information processing unit 20 failed while transmitting an analysis result to the host computer 62, correspondence between the transmission history and the reception history regarding the analysis result may not be established. That is, since the information processing unit 20 failed while transmitting the analysis result, the host computer 62 has not received the analysis result, and has a log indicating not having received the analysis result that corresponds to the measurement order information. On the other hand, there is a case where the failed information processing unit 20 has a log of having transmitted the analysis result. In this case, the alternate computer 5 having downloaded data of the failed information processing unit 20 from the external server 3 has a log of having transmitted the analysis result.

In the third modification, even when discrepancy in the transmission history and the reception history as described above has occurred between the alternate computer 5 and the host computer 62, sample measurement is performed after processing (data consistency check) for resolving the discrepancy is performed. It should be noted that the third modification can be combined with the first modification or the second modification.

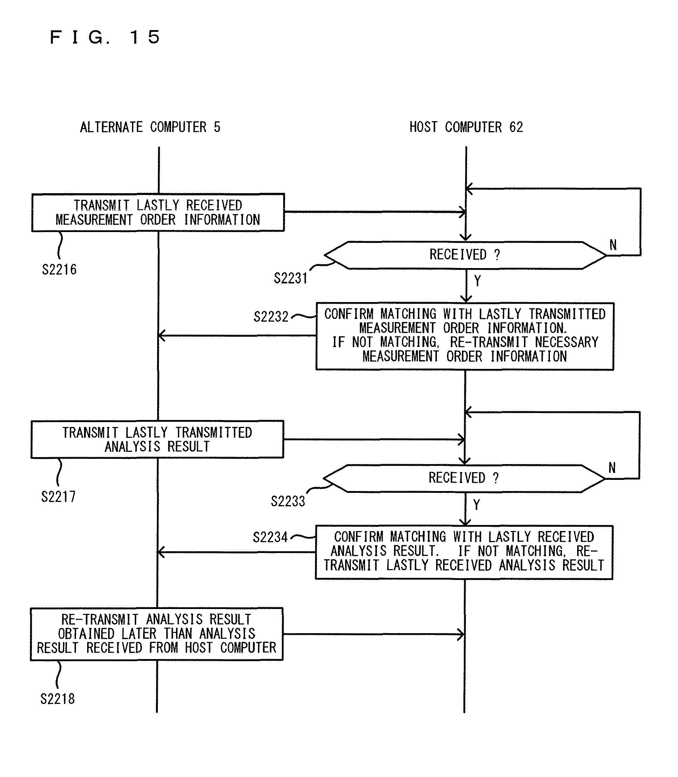

In the third modification, when the download program (such as control program) 84 has been installed in the alternate computer 5 (step S214 in FIG. 7), the alternate computer 5 transmits, as shown in FIG. 15, to the host computer 62, measurement order information (last measurement order information) corresponding to measurement order information lastly transmitted from the host computer 62 to the information processing unit 20, among a plurality of pieces of measurement order information having been downloaded from the first server 31 and stored in the storage section 52 (step S2216).

Upon receiving the "last measurement order information" (step S2231), the host computer 62 confirms whether the received "last measurement order information" matches the measurement order information that the host computer 62 itself recognizes as having lastly transmitted (step S2232). When they do not match each other, the host computer 62 transmits, to the alternate computer 5, measurement order information that the host computer 62 transmitted later than the "last measurement order information" received by the alternate computer 5 (step S2232). Accordingly, measurement order information stored in the host computer 62 and measurement order information stored in the alternate computer 5 match each other.

Subsequently, the alternate computer 5 transmits, to the host computer 62, an analysis result (last analysis result) corresponding to the analysis result lastly transmitted from the information processing unit 20 to the host computer 62, among a plurality of analysis results having been downloaded from the first server 31 and stored in the storage section 52 (step S2217).

Upon receiving the "last analysis result" (step S2233), the host computer 62 confirms whether the received "last analysis result" matches the analysis result that the host computer 62 itself recognizes as having lastly received from the information processing unit 20 (step S2234). When they do not match each other, the host computer 62 transmits, to the alternate computer 5, the analysis result that the host computer 62 itself recognizes as having lastly received (last analysis result on the host computer side) (step S2234).

Upon receiving the "last analysis result on the host computer side", the alternate computer 5 transmits, to the host computer 62, an analysis result that the information processing unit 20 transmitted later than the received "last analysis result on the host computer side" (step S2218). Accordingly, analysis results stored in the host computer 62 and analysis results stored in the alternate computer 5 match each other. It should be noted that, between the alternate computer 5 and the host computer 62, measurement order information or an analysis result itself may not be transmitted. Instead, a sample ID corresponding to measurement order information or an analysis result may be transmitted, whereby inquiry of the measurement order information or the analysis result may be made. Alternatively, a serial ID corresponding to measurement order information or an analysis result may be transmitted, whereby inquiry of the measurement order information or the analysis result may be made.

### [4.4 Additional notes]

The present invention is not limited to the above-described embodiments and modifications, and various modifications can be made. For example, the alternate computer 5 may also perform a part of the sample analysis processing. That is, a part of the sample analysis processing is performed by the alternate computer 5 and another part of the sample analysis processing may be performed by a virtual IPU 41b. Further, a processing result regarding a sample measured and analyzed by the alternate system may be provided with a flag so as to be differentiated from a processing result measured and analyzed during normal time. Accordingly, measurement data (raw data) of a sample measured and analyzed by the alternate system can be subjected to re-analysis afterwards, or an analysis result thereof can be examined afterwards.

Further, the download program 84 may include a data deletion program. The data deletion program is downloaded in the alternate computer 5. The data deletion program is programed such that when the new IPU 20 is connected to the system and the alternate system ends, the data deletion program automatically deletes the download program 84 stored in the alternate computer 5 and data (such as measurement data stored in the measurement data buffer 104) obtained while the alternate system was in operation. This can reduce the risk that unnecessary data or information leading to personal information will remain in the alternate computer 5.

Moreover, it is not necessary for the external server 3 to be provided in a facility (the data center 300) different from the facility 200 where the sample analyzer 2 is provided. The external server 3 may be provided in the same facility as the facility where the sample analyzer 2 is provided, and it is not necessary that the installation place of the external server 3 is outside the facility 200.

## Claims

1. A sample analysis method using a sample measuring apparatus configured to operate under control of a first computer, the first computer having at least a function of causing the sample measuring apparatus to start measurement of a sample, a function of receiving measurement data of the sample from the sample measuring apparatus, and a function of analyzing the received measurement data to generate an analysis result, the sample analysis method comprising:
making the sample measuring apparatus operate under control of a second computer instead of the first computer, the step including
downloading, from an external server, a control program for executing a function of causing the sample measuring apparatus to start measurement of a sample, a function of receiving measurement data of the sample from the sample measuring apparatus, and a function of transmitting the received measurement data to the external server, and
installing the control program in the second computer;
causing the second computer to execute the installed control program to cause the sample measuring apparatus to perform measurement of a sample, and to transmit measurement data received from the sample measuring apparatus to the external server; and
causing the external server to execute an analysis program for analyzing the measurement data received from the second computer, to analyze the measurement data and to generate an analysis result.

2. The sample analysis method of claim 1, comprising
causing the second computer to refer to the generated analysis result on the external server for displaying it.

3. The sample analysis method of claim 1 or 2, **characterized in that** making the sample measuring apparatus to operate under control of the second computer includes establishing a connection between the second computer in which the control program has been installed and the sample measuring apparatus.

4. The sample analysis method of any one of claims 1 through 3, comprising:
connecting the second computer with a host computer which manages analysis results; and
causing the external server to transmit the analysis result generated by the external server to the host computer.

5. The sample analysis method of any one of claims 1 through 4, **characterized in that**
the first computer is capable of storing therein data relating to measurement of a sample or analysis of measurement data, and
the method further comprises:
causing, while the sample measuring apparatus is capable of operating under control of the first computer, the first computer to transmit the data stored therein to the external server; and
causing the external server to store the data therein, to establish data synchronization of the data between the first computer and the external server.

6. The sample analysis method of claim 5, **characterized in that**
the data includes information for analysis to be used when the first computer analyzes the measurement data, and
the method comprises:
causing the external server to analyze, by using the information for analysis which had been transmitted from the first computer, the measurement data transmitted from the second computer.

7. The sample analysis method of claim 5 or 6, **characterized in that**
the data includes information for measurement to be used when the first computer controls measurement of a sample performed by the sample measuring apparatus, and
the method comprises:
causing the second computer to obtain, from the external server, the information for measurement stored in the external server, and to control, by using the obtained information for measurement, measurement of a sample performed by the sample measuring apparatus.

8. The sample analysis method of claim 7, **characterized in that**
causing the external server to provide the second computer with the control program and the information for measurement when the external server receives a request of downloading the control program from the second computer.

9. The sample analysis method of any one of claims 5 through 8, **characterized in that**
the data includes measurement order information indicating a measurement item to be measured for each of a plurality of samples, and
the method comprises:
causing the second computer to cause the sample measuring apparatus to start measurement of each sample based on the measurement order information stored in the external server.

10. The sample analysis method of claim 9, **characterized in that**
the sample measuring apparatus includes an ID obtaining part which obtains an ID from a sample container which contains a sample, and
the method comprises:
causing the second computer to cause the ID obtaining part of the sample measuring apparatus to obtain an ID, to receive the obtained ID from the sample measuring apparatus, and to transmit the received ID to the external server;
causing the external server to search, upon receiving the ID from the second computer, corresponding measurement order information by using the received ID as a key, and to transmit, to the second computer, the corresponding measurement order information or a measurement command based on the corresponding measurement order information; and
causing the second computer to control the sample measuring apparatus to start measurement of the sample, based on the received measurement order information or measurement command.

11. The sample analysis method of any one of claims 1 through 10, comprising:
causing the external server to transmit the data stored therein to a recovery computer;
making the sample measuring apparatus operate under control of the recovery computer instead of the second computer; and
causing the recovery computer to analyze measurement data generated in a state where the sample measuring apparatus operates under control of the recovery computer.

12. The sample analysis method of claim 11, **characterized in that**
the external server includes:
a first server capable of storing therein the data transmitted from the first computer and the data transmitted from the second computer; and
a second server which performs data synchronization of the data with the first server, and
the method further comprises:
causing, prior to replacement of the second computer with the recovery computer, the second server to stop performing the data synchronization of the data with the first server;
causing, upon stop of the data synchronization, the second server to transmit, to the recovery computer, the data transmitted from the first computer or the second computer and stored in the second server;
causing, even after the stop of the data synchronization, the first server to update the data stored in the first server, based on data communication with the second computer; and
causing, upon completion of transmission of the data stored in the second server to the recovery computer, the first server to transmit difference data, of the data stored in the first server, which has been updated after the stop of the data synchronization with the second server, to the recovery computer.

13. The sample analysis method of claim 4, **characterized in that**
the external server is capable of storing therein a data group including the data transmitted from the first computer and other data transmitted from another computer which controls another sample measuring apparatus different from the sample measuring apparatus, and
the method comprises:
causing the second computer to transmit, to the external server, data identification information for identifying the data transmitted from the first computer in the data group; and
causing the external server to extract the data transmitted from the first computer from the data group, based on the data identification information.

14. The sample analysis method of any one of claims 1 through 13, **characterized in that** the external server stores a control program group including the control program used in at least the second computer and a different type of control program for controlling measurement of a sample performed by a sample measuring apparatus of a type different from that of the sample measuring apparatus, and
the method further comprises:
causing the second computer to transmit, to the external server, program identification information for identifying the control program in the control program group; and
causing the external server to extract the control program from the control program group based on the program identification information, and to transmit the control program to the second computer.

15. A sample analysis system comprising:
a sample measuring apparatus including a means for measuring a sample;
a computer including a means for executing a control program for causing the sample measuring apparatus to measure a sample, and a communication program for performing communication with an external server; and
an external server including a means for storing data and connected to the computer via a network,
**characterized in that**
the computer functions as
a means for controlling the sample measuring apparatus to measure a sample and a means for receiving measurement data from the sample measuring apparatus by executing the control program, and
a means for transmitting the received measurement data to the external server by executing the communication program,
the external server functions by executing an analysis program as:
a means for analyzing the measurement data to generate an analysis result and
a means for storing the generated analysis result in the means for storing, and
the computer includes a means for displaying the analysis result by referring the same on the external server.
